# EUROPEAN PATENT APPLICATION

(11) **EP 4 825 956 A2**
(43) Date of publication of application: **21.10.2026**
(21) Application number: 26169432.7
(22) Date of filing: 31.03.2026
(51) Int. Cl.: C12M 1/107, C12M 1/12

(54) **METHOD FOR PRODUCING METHANE AND SYSTEM THEREFORE**

(30) Priority: 02.04.2025 DK PA202530205
(71) Applicant: Pura Futura A/S, 7100 Vejle (DK)
(72) Inventor: MIKKELSEN, Jeppe Hjuler, 8250 Egå (DK); ROSENLUND, Jørn, 6100 Haderslev (DK)
(74) Representative: Patentgruppen A/S

(57) **Abstract**

A method for producing methane is disclosed, the method comprising the steps of providing an aqueous solution, providing a packed bed reactor comprising a non-polymeric carrier material for microorganisms, the carrier material defining a carrier material zone, providing a hydrogen gas and an input gas comprising carbon dioxide to the packed bed reactor to convert at least part of the carbon dioxide to methane, and filling at least 10% by volume of said carrier material zone with said aqueous solution followed by removing at least part of said aqueous solution from the carrier material zone. A system for producing methane is also disclosed.

## Description

### FIELD OF THE INVENTION

The present invention relates to a method for producing methane according to the claims and a system for producing methane according to the claims.

### BACKGROUND OF THE INVENTION

In recent years, the production of methane has attracted increasing interest, e.g. in the form of biogas. However, raw biogas comprises substantial amounts of carbon dioxide, which has to be removed from the biogas. Therefore, biomethanation has been used to convert such carbon dioxide to increase the methane content in the biogas. Such biomethanation processes may even be used for other carbon dioxide sources.

However, since biomethanation relies on microorganisms, the conversion efficiency may be compromised when microorganisms are inactivated. This has led to specific reactors being designed to most effectively utilize and sustain the applied microorganisms. On the other hand, since the conversion relies on the use of hydrogen, which may not always be cost-effective, the overall process has several disadvantages.

It is the object of the present invention to solve the above problems.

### SUMMARY OF THE INVENTION

The invention relates to a method for producing methane comprising the steps of providing an aqueous solution, providing a packed bed reactor comprising a non-polymeric carrier material for microorganisms, the carrier material defining a carrier material zone, providing a hydrogen gas and an input gas comprising carbon dioxide to the packed bed reactor to convert at least part of the carbon dioxide to methane, and filling at least 10% by volume of said carrier material zone with said aqueous solution followed by removing at least part of said aqueous solution from the carrier material zone.

One advantage of the invention may be that the invention facilitates increased efficiency when producing methane. Typically, conventional methane production in a packed bed reactor utilizes liquid continuously being introduced into the reactor, e.g. by trickling. Hereby, channeling may occur, i.e. where fluid flow such as the gas flow and/or a trickling fluid flow is unevenly distributed and concentrated in channels, whereby the efficiency of the methane production may be reduced. Additionally, conventional methane production in a packed bed reactor suffers from impurities such as acids, which may harm/affect microorganisms producing methane. However, the present invention advantageously facilitates a method for producing methane, which not only may provide a way to increase efficiency in the methane production, by avoiding channeling and removing impurities, but also facilitate a way for maintaining efficiency and increasing safety.

In more detail, by filling at least 10% by volume of the carrier material zone with said aqueous solution, the non-polymeric carrier material may be inoculated and impurities may be removed to improve the efficiency of at least a part of the carrier material zone being filled. Applying a non-polymeric carrier material facilitates good adherence of microorganisms and at the same time e.g. reconfiguration of the carrier material by filling the carrier material zone at least partly by the aqueous solution, which may then, together with inoculation or re-inoculation and with impurity removal, facilitate improved conversion of carbon dioxide to methane.

Thus, an advantage of the invention may be that a more dynamic method may be obtained, in particular a method which may be efficient even when going through a cycle of repeatedly turning on and off, e.g. in response to whether cost efficient conversion may be available. Normally, the turning on and off of the conversion is associated with a period of inefficient conversion. However, the present invention facilitates an efficient conversion, e.g. by allowing re-inoculation of the carrier material, removal of impurities/inhibitors, and repacking of the carrier material to decrease channeling. This facilitates overcoming the disadvantages of having to frequently turn the process on and off and thereby provides a more dynamic conversion method.

In the present context, the term "carrier material zone" refers to the zone defined by the carrier material during normal operation of the packed bed reactor. While the carrier material in some embodiments may move at least partly out of the carrier material zone if filling the packed bed reactor with sufficient amounts of aqueous solution, this does not contribute to the definition of the carrier material zone.

In the present context, it should be understood that the filling and removing are performed in the recited order, i.e. by first filling at least 10% by volume of said carrier material zone with said aqueous solution and then removing at least part of said aqueous solution from the carrier material zone. In some embodiments, the filling and removing is performed before the step of providing a hydrogen gas and an input gas comprising carbon dioxide to the packed bed reactor to convert at least part of the carbon dioxide to methane. In some embodiments, the filling and removing is performed after the step of providing a hydrogen gas and an input gas comprising carbon dioxide to the packed bed reactor to convert at least part of the carbon dioxide to methane.

In an embodiment of the invention, the step of filling and removing is before the step of converting carbon dioxide to methane.

In an embodiment of the invention, the step of filling and removing is after the step of converting carbon dioxide to methane.

In an embodiment of the invention, the conversion step is carried out before the step of filling and removing and after the step of filling and removing.

In an embodiment of the invention, the conversion step and the step of filling and removing are carried out sequentially, e.g. with multiple repetitions.

In an embodiment of the invention, said aqueous solution is selected from the group consisting of wastewater, digestate, agricultural residues such as manure components, synthetic mediums, aqueous fertilizer, water such as microbially produced metabolic water, and any combination thereof.

An advantage of the above embodiment may be that utilization of the aqueous solution may reduce the overall cost for the biogas/biomethane production and make the production more environmentally friendly.

In an embodiment of the invention, said aqueous solution comprises wastewater.

In an embodiment of the invention, said aqueous solution comprises manure components.

An advantage of the above embodiment may be that by utilizing manure components microorganisms for biogas/biomethane production can be introduced. Further, it may provide nutrients for the microorganisms. Thus, it may reduce the overall cost for the biogas/biomethane production and make the production more environmentally friendly.

In an embodiment of the invention, said aqueous solution comprises microorganisms, such as microorganisms for biogas and/or biomethane production.

An advantage of the above embodiment may be that filling the carrier material zone with aqueous solution comprising microorganisms provides a way to inoculate the carrier material hereby establishing a stable microbial population, restoring optimal digestion conditions and thus increasing the efficiency of producing methane.

According to an embodiment of the invention, the subsequent removal of said aqueous solution is conducted by introducing a gas, such as said hydrogen gas and/or said input gas.

In an embodiment of the invention, the step of filling and removing reduces the content of impurities in the packed bed reactor.

An advantage of the above embodiment may be that by removing impurities, the conditions for the microorganisms may improve and enhance the methane production and conversion rates thereof.

In an embodiment of the invention, said impurities comprise acids, such as volatile fatty acids such as acetic acid, propionic acid, and butyric acid.

According to an embodiment of the invention, said impurities comprise accumulated biomass.

According to an embodiment of the invention, said impurities comprise inhibitors.

In an embodiment of the invention, said packed bed reactor comprises trickling.

An advantage of the above embodiment may be that trickling in a bioreactor enhances microbial activity and overall system efficiency. Trickling improves contact between microorganisms and nutrients and/or substrates, thereby promoting better methane production in anaerobic systems. Further, the continuous flow of liquid provided by trickling enhances mass transfer, ensuring even distribution of nutrients. Additionally, it may prevent overheating in a reactor, maintaining the proper conditions for microorganisms.

In an embodiment of the invention, the packed bed reactor is a trickle bed reactor.

In a trickle bed reactor, liquid is typically distributed over the carrier material, which may lead to uneven distribution and channeling over time. The step of filling and removing may advantageously address such channeling by redistributing the carrier material and restoring even liquid distribution across the carrier material zone.

It should be noted that the time and interval of the trickling vary greatly depending on the efficiency and productivity of the bioreactor. In some instances, the interval and time could be reduced to decrease the liquid film on top of the microorganisms, which would further result in a greater mass transfer of hydrogen from gas to liquid.

In an embodiment of the invention, said trickling is conducted with said aqueous solution.

In an embodiment of the invention, the step of converting at least part of the carbon dioxide to methane is conducted in a gas fermentation process.

In an embodiment of the invention, said gas fermentation process comprises microorganisms, such as bacteria and/or archaea.

In an embodiment of the invention, said gas fermentation process comprises microorganisms selected from the group consisting of methanogens, acetogens, and any combination thereof.

According to an embodiment of the invention, the hydrogen gas is supplied directly from said hydrogen generator to the gas fermentation process.

In the present context, the term "hydrogen generator" may e.g. refer to an electrolyzer.

As used herein, the term "carrier material" is understood as a carrier for microorganisms which is compatible with microorganisms such as methanogens in the setting of biomethanation. The carrier material for microorganisms is solid and may be porous substance. Also, the carrier material is a non-polymeric material, such as an inorganic material. The carrier material provides a suitable surface or matrix for the attachment, growth, and survival of microorganisms in various biological processes, such as biomethanation. It is noted that the carrier material may sometimes also be referred to as packing material.

An advantage of using a non-polymeric carrier material may be improved thermal stability, chemical resistance, and structural integrity under the conditions present in the packed bed reactor. Non-polymeric materials such as vulcanized ash, zeolites, glass foam, ceramics, expanded clay, perlite, and pumice may provide superior biofilm adhesion properties compared to polymeric alternatives.

In an embodiment of the invention, the carrier material is not a spongy material.

In an embodiment of the invention, said carrier material is porous.

An advantage of the above embodiment may be that the carrier material can increase the effective surface area for growth of said microorganisms. Thus, increasing the efficiency of the method for producing methane. In more detail, the porosity of the carrier material may contribute to floatability of the carrier material, thereby facilitating reconfiguration of carrier material after filling the packed bed reactor. Also, the porosity may contribute to water retention of the carrier material.

In an embodiment of the invention, the carrier material has a void fraction of at least 20%, such as at least 30%.

In an embodiment of the invention, the carrier material has a void fraction of 20 to 90%, such as 30 to 70%.

In the above embodiment, the term void fraction refers to the material characteristic. Of course, if filling a volume with incoherent particles material, a certain void fraction of the volume may be defined. However, the void fraction of the above embodiment refers to the material itself, i.e. to define the porosity of individual particles of carrier material.

In an embodiment of the invention, the carrier material has a specific surface area of at least 400 m2/m3, such as at least 500 m2/m3.

In an embodiment of the invention, the carrier material has a specific surface area of 400 to 3000 m2/m3, such as 500 to 1500 m2/m3.

In an embodiment of the invention, the carrier material has a water holding capacity of at least 10%, such as at least 20%.

In an embodiment of the invention, the carrier material has a water holding capacity of 10-80%, such as 10-60%, such as 20-50%.

As used in the above embodiment, the water holding capacity refers to the weight percentage increase of the carrier material after first submerging the carrier material for a suitable time period, such as several hours, and then allowing the carrier material to drain, e.g. in a sieve or mesh, until no further water drains from the carrier material and comparing that weight to an oven-dried carrier materials.. Preferably, the draining is performed in a humid environment to avoid drying any part of the carrier material. In an embodiment, the water holding capacity is measured in accordance with the Danish standard DS/EN 1097-6:2022.

In an embodiment of the invention, the carrier material is an inorganic material.

An advantage of the above embodiment may be that the inorganic carrier material may combine a good resilience with good biofilm adhesiveness, thereby facilitating long lasting efficiency.

In an embodiment of the invention, the carrier material is selected from the group consisting of biochar, vulcanized ash, zeolites, glass foam, ceramics, clay such as expanded clay, perlite, pumice, and any combination thereof.

In an embodiment of the invention, the carrier material is selected from the group consisting of vulcanized ash, zeolites, glass foam, ceramics, clay such as expanded clay, perlite, pumice, and any combination thereof.

In an embodiment of the invention, the carrier material is selected from the group consisting of vulcanized ash, zeolites, glass foam, ceramics, expanded clay, perlite, and any combination thereof.

In an embodiment of the invention, the carrier material is vulcanized ash. In an embodiment of the invention, the carrier material is zeolites. In an embodiment of the invention, the carrier material is glass foam. In an embodiment of the invention, the carrier material is a ceramic. In an embodiment of the invention, the carrier material is expanded clay. In an embodiment of the invention, the carrier material is perlite.

In an embodiment of the invention, the carrier material is an aggregate, such as a lightweight aggregate.

In an embodiment of the invention, the carrier material is non-porous.

In an embodiment of the invention, said carrier material increases the number of microorganisms in said packed bed reactor.

In an embodiment of the invention, the carrier material is floatable.

An advantage of the above embodiment may be that the carrier material may be reconfigured after filling the packed bed reactor with said aqueous solution, which may facilitate increased efficiency of the methane conversion.

In an embodiment of the invention, the carrier material is floatable, and the step of filling at least 10% by volume of the carrier material zone with the aqueous solution causes at least part of the carrier material to float, thereby reconfiguring the orientation and/or position of the carrier material.

Upon removing the aqueous solution, the carrier material settles in a new configuration, which may reduce or eliminate channeling that has developed during operation. This reconfiguration may be particularly advantageous in trickle bed reactors where channeling may develop over time due to the continuous trickling of liquid over the carrier material.

It is noted that in the present context the term "floatable" refers to the material being floatable in water, which may be facilitated by using a material with a bulk density below the density of water and or a porous material utilizing gas pockets to obtain an effective density below that of water.

In an embodiment of the invention, said hydrogen gas is provided by a chemical reaction such as electrolysis, biomass gasification, pyrolysis, coal gasification, fermentation or partial oxidation of hydrocarbons.

In an embodiment of the invention, said hydrogen gas is provided by an electrochemical reaction, such as electrolysis.

According to an embodiment of the invention, hydrogen gas is produced from an electrical source.

Thus, according to an embodiment of the electrical source enables production of hydrogen gas from a renewable source such as electricity. As a result, the methane production utilizing hydrogen from a renewable source of energy, can be categorized and certified as e-methane.

An advantage of the above embodiment is that it acts as an energy carrier to store excess renewable electricity.

According to an embodiment of the invention, hydrogen gas is produced as a byproduct from a chemical process.

According to an embodiment of the invention, said electrochemical reaction is conducted under non-alkaline conditions.

According to an embodiment of the invention, said electrochemical reaction is proton exchange membrane electrolysis.

According to an embodiment of the invention, said hydrogen gas comprises hydrogen in an amount of at least 90%, such as hydrogen in an amount of at least 93%, such as a hydrogen in an amount of at least 96%, such as hydrogen in an amount of at least 99%.

In an embodiment of the invention, the input gas comprises raw biogas.

An advantage of the above embodiment may be that upgrading of the raw biogas may be facilitated, particularly by converting at least of the carbon dioxide to additional methane. Since raw biogas comprises both methane and carbon dioxide, it is conventionally processed to remove carbon dioxide to obtain a very high concentration of methane. However, in the above embodiment, the carbon dioxide is not only at least partly removed, it is even at least partly converted to methane.

According to an embodiment of the invention, the input gas is raw biogas.

As used herein, the term "raw biogas" relates to gas produced through anaerobic digestion of organic matter by microorganisms in the absence of oxygen. This process breaks down substrates such as agricultural waste, manure, food residues, sewage, or other biodegradable materials. The resulting raw biogas mixture comprises methane, carbon dioxide, but may also comprise less amounts of other gases such as hydrogen sulfide and water vapor.

According to an embodiment of the invention, the raw biogas comprises a mixture of methane and carbon dioxide.

According to an embodiment of the invention, the raw biogas comprises a mixture of methane, carbon dioxide, and further components, such as one or more of nitrogen, hydrogen, hydrogen sulfide, water and oxygen.

**In** an embodiment of the invention, the input gas has a content of carbon dioxide of at least 5% by weight of the input gas, such as at least 10% by weight of the input gas, such as at least 15% by weight of the input gas, such as at least 20% by weight of the input gas, such as at least 25% by weight of the input gas, such as at least 30% by weight of the input gas.

According to an embodiment of the invention, the input gas has a content of carbon dioxide of 5 to 75% by weight of the input gas, such as 10 to 70% by weight of the input gas, such as 15 to 65% by weight of the input gas, such as 20 to 60% by weight of the input gas, such as 25 to 55% by weight of the input gas, such as 35 to 50% by weight of the input gas, such as 40 to 45% by weight of the input gas.

In an embodiment of the invention, the input gas and the hydrogen gas are fed into the packed bed reactor below the carrier material zone.

One advantage of the above embodiment may be an improved efficiency of the packed bed reactor, in particular due to an improved inoculation of the carrier material and/or an improved removal of impurities and inhibitors in the carrier zone. When feeding the gasses to be converted, i.e. the input gas and the hydrogen gas, into the packed bed reactor below the carrier material zone, the concentration of these yet to be converted gasses would decrease throughout the carrier material zone as conversion occurs. Therefore, the highest concentration of unconverted gasses would be in the lower part of the carrier material zone, also leading to the highest microbial activity being located in this part of the carrier material zone. If only a part of the carrier material zone is filled by the aqueous, feeding the input gas and the hydrogen gas from the bottom would increase the overlap between the part of the carrier material zone being filled with aqueous liquid (aqueous solution) and the high activity part of the carrier material zone.

In an embodiment of the invention, at least 5% of said carbon dioxide is converted into methane, such as at least 10% of said carbon dioxide is converted into methane, such as at least 20% of said carbon dioxide is converted into methane, such as at least 30% of said carbon dioxide is converted into methane, such as at least 40% of said carbon dioxide is converted into methane.

An advantage of the above embodiment may be that the overall product may be purer by providing this upgrade of methane. Thus, the above embodiment may reduce the impurities and make the produced gas comparable to naturally occurring methane. Furthermore, upgrading inherent impurities may further reduce the costs of purification and provide a more environmentally friendly solution for producing methane.

According to an embodiment of the invention, 5 to 100% of said carbon dioxide is converted into methane, such as 10 to 80% of said carbon dioxide is converted into methane, such as 15 to 60% of said carbon dioxide is converted into methane, such as 20 to 40% of said carbon dioxide is converted into methane.

In an embodiment of the invention, at least 50% of said hydrogen gas is converted into methane, such as at least 60% of said hydrogen gas is converted into methane, such as at least 70% of said hydrogen gas is converted into methane, such as at least 80% of hydrogen gas is converted into methane, such as at least 90% of said hydrogen gas is converted into methane.

In an embodiment of the invention, said flow of the aqueous solution into the packed bed reactor is configured to fill at least 15% by volume of said carrier material zone with said aqueous solution, such as at least 20% by volume of said carrier material zone, such as at least 25% by volume of said carrier material zone, such as at least 30% by volume of said carrier material zone.

According to an embodiment of the invention, said flow of the aqueous solution into the packed bed reactor is configured to fill at least 15 to 50% by volume of said carrier material zone with said aqueous solution, such as at least 20 to 45% by volume of said carrier material zone, such as least 25 to 40% by volume of said carrier material zone, such as at least 30 to 35% by volume of said carrier material zone.

An advantage of the above embodiment may be that filling the carrier material zone with aqueous solution provides a way to remove impurities, such as acids, when producing methane. Thus, by e.g., removing acids extends the lifespan of gas storage facilities and metal components. Furthermore, impurities may harm/affect microorganisms producing methane. Hence, removal of impurities may thus increase the efficiency of producing methane.

A further advantage of the above embodiment may be that filling the carrier material zone with aqueous solution provides a way to repack the carrier material and avoid channeling of fluids. In e.g., packed bed reactors, channeling can cause underutilization of active surfaces, reducing reaction efficiency. Thus, by avoiding channeling, microorganisms may get increased exposure to nutrients, further increasing the efficiency of producing methane.

A further advantage of the above embodiment may be that filling the carrier material zone with aqueous solution, comprising microorganisms, provides a way to inoculate the carrier material hereby establishing a stable microbial population, restoring optimal digestion conditions and thus increasing the efficiency of producing methane.

In an embodiment of the invention, said flow of the aqueous solution into the packed bed reactor is configured to fill at least 75% by volume of said carrier material zone with said aqueous solution, such as at least 85% by volume of said carrier material zone, such as at least 90% by volume of said carrier material zone, such as at least 95% by volume of said carrier material zone, such as 100% by volume of said carrier material zone.

An advantage of the above embodiment may be that the carrier material may be disturbed to reconfigure the orientation and/or position of the carrier material, whereby channeling may be reduced or eliminated. In other words, the carrier material may be repacked to obtain a new configuration. This may be especially advantageous when using floatable carrier material, which may facilitate the reconfiguration of the carrier material.

According to an embodiment of the invention, said flow of the aqueous solution into the packed bed reactor is configured to fill at least 50 to 95% by volume of said carrier material zone with said aqueous solution, such as at least 55 to 90% by volume of said carrier material zone, such as at least 60 to 85% by volume of said carrier material zone, such as at least 65 to 80% by volume of said carrier material zone.

According to an embodiment of the invention, said flow of the aqueous solution into the packed bed reactor is configured to fill at least 15 to 95% by volume of said carrier material zone with said aqueous solution, such as least 25 to 85% by volume of said carrier material zone, such as at least 35 to 75% by volume of said carrier material zone with, such as at least 45 to 65% by volume of said carrier material zone.

In an embodiment of the invention, said flow of the aqueous solution into the packed bed reactor is configured to fill at least 90% by volume of said packed bed reactor with said aqueous solution, such as at least 95% by volume of said packed bed reactor, such as at least 98% by volume of said packed bed reactor, such as 100% by volume of said packed bed reactor.

An advantage of the above embodiment may be that filling the carrier material zone with aqueous solution provides a way to displace gases comprised in the carrier material zone. Thus, a gaseous environment that could disrupt microbial digestion is avoided. Furthermore, gas accumulation, which can lead to explosion risks, is avoided. Thus, the above embodiment provides a way to maintain efficiency and safety.

In an embodiment of the invention, filling and removing step comprises removing at least 5% of said aqueous solution from the carrier material zone, such as at least 15% from said carrier material zone, such as at least 30% from said carrier material zone, such as at least 50% from said carrier material zone, such as at least 75% from said carrier material zone, such as 100% from said carrier material zone.

In an embodiment of the invention, the filling is achieved by establishing a flow of the aqueous solution into the packed bed reactor, e.g. by using a pump arrangement.

In an embodiment of the invention, the removing is achieved by establishing a flow of the aqueous solution out of the packed bed reactor, e.g. by using a pump arrangement.

In an embodiment of the invention, the packed bed reactor has an interior atmosphere comprising oxygen before or after the step of filling and removing.

The above embodiment may be especially advantageous for maintenance with improved safety. Maintenance constitutes a problem since the internal atmosphere of the packed bed reactor after conversion comprises methane, and when opening the packed bed reactor, this will lead to oxygen being led into the internal atmosphere and mixing with the methane. Such mixture of oxygen and methane naturally constitutes a significant safety problem. Similarly, when maintenance is concluded and conversion starts again, production of methane will again lead to an internal atmosphere comprising both methane and oxygen. However, by filling at least a significant part of the packed bed reactor, as in the above embodiment, the internal atmosphere is eliminated or nearly eliminated, thereby significantly increasing safety.

In an embodiment of the invention, the packed bed reactor has an interior atmosphere comprising oxygen before the step of filling and removing.

In an embodiment of the invention, the packed bed reactor has an interior atmosphere comprising oxygen after the step of filling and removing.

The invention further relates to a system for producing methane, the system comprising a packed bed reactor comprising a non-polymeric carrier material for microorganisms, the carrier material defining a carrier material zone, an aqueous solution source connected to the packed bed reactor, a flow controlling arrangement such as a pump arrangement, a hydrogen gas inlet connected to the packed bed reactor, and an input gas inlet connected to the packed bed reactor, the input gas comprising carbon dioxide, wherein the flow controlling arrangement is configured to establish a flow of aqueous solution from said aqueous solution source into said packed bed reactor to fill at least 10% by volume of the carrier material zone, and to establish a flow of aqueous solution from said packed bed reactor to remove at least part of said aqueous solution from the carrier material zone, and wherein the packed bed reactor is configured to convert at least part of the carbon dioxide to methane.

In the present context the term "flow controlling arrangement" refers to an arrangement controlling the flow of the aqueous solution. The flow controlling arrangement may comprise a pump arrangement and/or one or more controllable valves.

In the present context the term "pump arrangement" refers to one or more pumps. In some embodiments, the pump arrangement is a single pump arranged to establish a flow of aqueous solution into the packed bed reactor and also arranged to establish a flow of aqueous solution out from the packed bed reactor. In some embodiments, the pump arrangement comprises at least a first pump arranged to establish a flow of aqueous solution into the packed bed reactor and at least a second pump arranged to establish a flow of aqueous solution out from the packed bed reactor.

In an embodiment of the invention, the system is configured to operate in accordance with the invention or any of its embodiments.

The invention further relates to aspects and embodiments according to the following clauses.

Clause 1. A method for producing methane (MET) comprising the steps of providing an aqueous solution (AS),
providing a packed bed reactor (PBR) comprising a non-polymeric carrier material for microorganisms, the carrier material defining a carrier material zone (CMZ),
providing a hydrogen gas (HG) and an input gas (IG) comprising carbon dioxide to the packed bed reactor (PBR) to convert at least part of the carbon dioxide to methane (MET), and
filling at least 10% by volume of said carrier material zone (CMZ) with said aqueous solution (AS) followed by removing at least part of said aqueous solution (AS) from the carrier material zone (CMZ).

Clause 2. The method according to clause 1, wherein the step of filling and removing is before the step of converting carbon dioxide to methane (MET).

Clause 3. The method according to clause 1 or 2, wherein the step of filling and removing is after the step of converting carbon dioxide to methane (MET).

Clause 4. The method according to any of clauses 1-3, wherein said aqueous solution (AS) is selected from the group consisting of wastewater, digestate, agricultural residues such as manure components, synthetic mediums, aqueous fertilizer, water such as microbially produced metabolic water, and any combination thereof.

Clause 5. The method according to any of clauses 1-4, wherein said aqueous solution (AS) comprises microorganisms, such as microorganisms for biogas and/or biomethane production.

Clause 6. The method according to any of clauses 1-5, wherein the step of filling and removing reduces the content of impurities in the packed bed reactor (PBR).

Clause 7. The method according to any of clauses 1-6, wherein said impurities comprise acids, such as volatile fatty acids such as acetic acid, propionic acid and butyric acid.

Clause 8. The method according to any of clauses 1-7, wherein said packed bed reactor (PBR) comprises trickling.

Clause 9. The method according to any of clauses 1-8, wherein said trickling is conducted with said aqueous solution.

Clause 10. The method according to any of clauses 1-9, wherein the step of converting at least part of the carbon dioxide to methane (MET) is conducted in a gas fermentation process.

Clause 11. The method according to any of clauses 1-10, wherein said gas fermentation process comprises microorganisms, such as bacteria and/or archaea.

Clause 12. The method according to any of clauses 1-11, wherein said gas fermentation process comprises microorganisms selected from the group consisting of methanogens, acetogens, and any combination thereof.

Clause 13. The method according to any of clauses 1-12, wherein said carrier material is porous.

Clause 14. The method according to any of clauses 1-13, wherein the carrier material has a void fraction of at least 20%, such as at least 30%.

Clause 15. The method according to any of clauses 1-14, wherein the carrier material has a specific surface area of at least 400 m2/m3, such as at least 500 m2/m3.

Clause 16. The method according to any of clauses 1-15, wherein the carrier material has a water holding capacity of at least 10%, such as at least 20%.

Clause 17. The method according to any of clauses 1-16, wherein the carrier material is an inorganic material.

Clause 18. The method according to any of clauses 1-17, wherein the carrier material is selected from the group consisting of biochar, vulcanized ash, zeolites, glass foam, ceramics, clay such as expanded clay, perlite, pumice, and any combination thereof.

Clause 19. The method according to any of clauses 1-18, wherein said carrier material increases the number of microorganisms in said packed bed reactor (PBR).

Clause 20. The method according to any of clauses 1-19, wherein the carrier material is floatable.

Clause 21. The method according to any of clauses 1-20, wherein said hydrogen gas (HG) is provided by a chemical reaction such as electrolysis, biomass gasification, pyrolysis, coal gasification, fermentation or partial oxidation of hydrocarbons.

Clause 22. The method according to any of clauses 1-21, wherein said hydrogen gas (HG) is provided by an electrochemical reaction, such as electrolysis.

Clause 23. The method according to any of clauses 1-22, wherein the input gas (IG) comprises raw biogas.

Clause 24. The method according to any of clauses 1-23, wherein the input gas (IG) has a content of carbon dioxide of at least 5% by weight of the input gas, such as at least 10% by weight of the input gas, such as at least 15% by weight of the input gas, such as at least 20% by weight of the input gas, such as at least 25% by weight of the input gas, such as at least 30% by weight of the input gas.

Clause 25. The method according to any of clauses 1-24, wherein the input gas (IG) and the hydrogen gas (HG) are fed into the packed bed reactor (PBR) below the carrier material zone (CMZ).

Clause 26. The method according to any of clauses 1-25, wherein at least 50% of said hydrogen gas (HG) is converted into methane (MET), such as at least 60% of said hydrogen gas (HG) is converted into methane (MET), such as at least 70% of said hydrogen gas (HG) is converted into methane (MET), such as at least 80% of hydrogen gas (HG) is converted into methane (MET), such as at least 90% of said hydrogen gas (HG) is converted into methane (MET).

Clause 27. The method according to any of clauses 1-26, wherein said flow of the aqueous solution (AS) into the packed bed reactor (PBR) is configured to fill at least 15% by volume of said carrier material zone (CMZ) with said aqueous solution, such as at least 20% by volume of said carrier material zone (CMZ), such as at least 25% by volume of said carrier material zone (CMZ), such as at least 30% by volume of said carrier material zone (CMZ).

Clause 28. The method according to any of clauses 1-27, wherein said flow of the aqueous solution (AS) into the packed bed reactor (PBR) is configured to fill at least 75% by volume of said carrier material zone (CMZ) with said aqueous solution (AS), such as at least 85% by volume of said carrier material zone (CMZ), such as at least 90% by volume of said carrier material zone (CMZ), such as at least 95% by volume of said carrier material zone (CMZ), such as 100% by volume of said carrier material zone (CMZ).

Clause 29. The method according to any of clauses 1-28, wherein said flow of the aqueous solution (AS) into the packed bed reactor (PBR) is configured to fill at least 90% by volume of said packed bed reactor (PBR) with said aqueous solution (AS), such as at least 95% by volume of said packed bed reactor (PBR), such as at least 98% by volume of said packed bed reactor (PBR), such as 100% by volume of said packed bed reactor (PBR).

Clause 30. The method according to any of clauses 1-29, wherein filling and removing step comprises removing at least 5% of said aqueous solution (AS) from the carrier material zone (CMZ), such as at least 15% from said carrier material zone (CMZ), such as at least 30% from said carrier material zone (CMZ), such as at least 50% from said carrier material zone (CMZ), such as at least 75% from said carrier material zone (CMZ), such as 100% from said carrier material zone (CMZ).

Clause 31. The method according to any of clauses 1-30, wherein the filling is achieved by establishing a flow of the aqueous solution (AS) into the packed bed reactor (PBR), e.g. by using a pump arrangement.

Clause 32. The method according to any of clauses 1-31, wherein the removing is achieved by establishing a flow of the aqueous solution (AS) out of the packed bed reactor (PBR), e.g. by using a pump arrangement.

Clause 33. The method according to any of clauses 1-32, the packed bed reactor has an interior atmosphere comprising oxygen before or after the step of filling and removing.

Clause 34. The method according to any of clauses 1-33, the packed bed reactor is a trickle bed reactor.

Clause 35. A system for producing methane (MET), the system comprising
- a packed bed reactor (PBR) comprising a non-polymeric carrier material for microorganisms, the carrier material defining a carrier material zone (CMZ),
- an aqueous solution source (ASS) connected to the packed bed reactor (PBR),
- a flow controlling arrangement (FCA) such as a pump arrangement,
- a hydrogen gas inlet (GI) connected to the packed bed reactor (PBR), and
- an input gas inlet (GI) connected to the packed bed reactor (PBR), the input gas (IG) comprising carbon dioxide,

wherein the flow controlling arrangement (FCA) is configured to establish a flow of aqueous solution (AS) from said aqueous solution source (ASS) into said packed bed reactor (PBR) to fill at least 10% by volume of the carrier material zone (CMZ), and
   to establish a flow of aqueous solution (AS) from said packed bed reactor (PBR) to remove at least part of said aqueous solution (AS) from the carrier material zone (CMZ), and
wherein the packed bed reactor (PBR) is configured to convert at least part of the carbon dioxide to methane (MET).

Clause 36. The system according to clause 35, wherein the system is configured to operate in accordance with the method according to any of clauses 1-34.

### FIGURES

The invention will now be described with reference to the figures, where
Figure 1 illustrates a method of producing methane to an embodiment of the invention,
Figures 2a-2b illustrate a method of producing methane according to an embodiment of the invention,
Figures 3a-3c illustrate a method of producing methane according to an embodiment of the invention,
Figure 4 illustrates a method of producing methane to an embodiment of the invention, and
Figure 5 illustrates a system for producing methane according to an embodiment of the invention.

### DETAILED DESCRIPTION

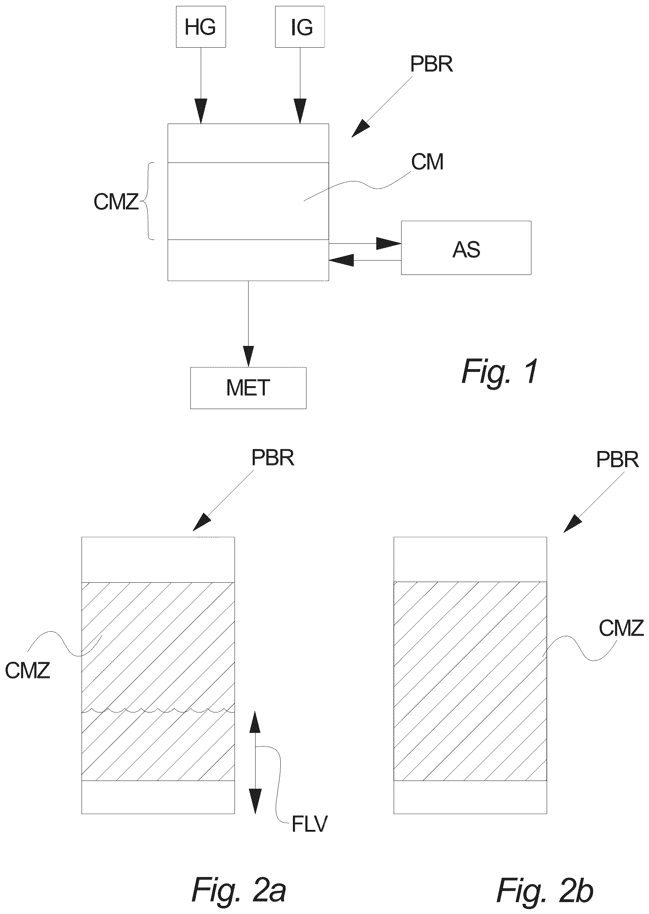

Referring to figure 1, a method for producing methane is illustrated according to an embodiment of the invention.

The method is carried out in a packed bed reactor PBR, which comprises a non-polymeric carrier material CM for microorganisms. The carrier material CM defines a carrier material zone CMZ, i.e. the zone spanned by the carrier material CM when the packed bed reactor PBR is not filled with aqueous solution.

In addition to providing the packed bed reactor PBR, the method also involves providing an aqueous solution AS. Various aqueous solutions as described herein are usable within the embodiment of figure 1.

The method further comprises the step of providing a hydrogen gas HG and an input gas IG comprising carbon dioxide to the packed bed reactor PBR. It is noted that the hydrogen gas HG and the input gas IG may be delivered separately to the reactor chamber of the packed bed reactor, or they may be mixed prior to injection into the reaction chamber, e.g. by delivery to a by common gas receiving connector for both the hydrogen gas and the input gas. Thereby, at least part of the carbon dioxide is converted to methane. This step may be referred to as the conversion step.

The method further comprises filling at least 10% by volume of said carrier material zone CMZ with said aqueous solution AS, also referred to as filling or the filling step. The degree of filling may be referred to as the filling level, Following the filling, the method comprises removing at least part of said aqueous solution AS from the carrier material zone CMZ, also referred to as the removing step or simply removing.

Figure 1 illustrates the aqueous solution AS as being removed to the same source as being filled from, however, the aqueous solution may also be removed to a different target than the initial source.

It is noted that within the embodiment of figure 1, the filling and removing may be before the conversion step, or the filling and removing may be after the conversion step.

Referring now to figures 2a and 2b, an embodiment of the invention is illustrated. This embodiment is applicable within the embodiment of figure 1.

Figure 2a illustrates an embodiment where an aqueous solution AS is filled up to a certain level, which may be referred to as the filling level FLV. In some embodiments, it may be given as the percentage of the carrier material zone CMZ being filled. In some embodiments, the aqueous solution AS may fill the entire carrier material zone CMZ or even up to the entire packed bed reactor PBR.

Then, the aqueous solution AS is removed from the packed bed reactor PBR. In some embodiments the entire aqueous solution AS is removed. In some embodiments, an amount of aqueous solution AS remains in the packed bed reactor PBR, typically below the carrier material zone CMZ.

After removing the aqueous solution AS from the packed bed reactor PBR, the packed bed reactor PBR, as illustrated in figure 2b, is ready for the conversion step. The conversion step is carried out as explained in relation to the embodiment of figure 1.

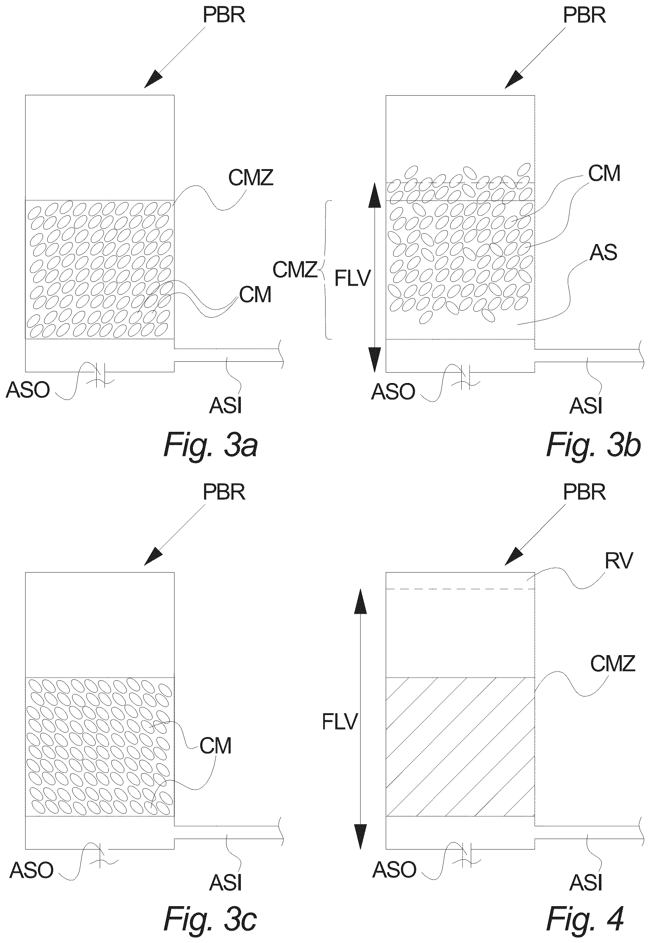

Referring now to figures 3a-3c, an embodiment of the invention is illustrated.

In figures 3a-3c, a packed bed reactor PBR is illustrated. It is noted that the packed bed reactor PBR may operate in accordance with the embodiments illustrated in figure 1. Therefore, e.g. hydrogen gas HG, input gas IG, and methane MET are not illustrated in figures 3a-3c. The packed bed reactor PBR comprises an aqueous solution inlet ASI for establishing a flow of aqueous solution into the packed bed reactor PBR. The packed bed reactor PBR further comprises an aqueous solution outlet ASO for establishing a flow of aqueous solution out of the packed bed reactor PBR.

In more detail, figures 3a-3c illustrate the step of filling and removing. First, as shown in figure 3a, the carrier material CM, defining the carrier material zone CMZ, is in a first configuration.

Then, after establishing a flow of aqueous solution AS through the aqueous solution inlet ASI into the packed bed reactor PBR, which is filled to a filling level FLV, the carrier material CM is floating in the aqueous solution AS, as illustrated in figure 3b. The orientation of the individual carrier material particles are thereby at least partly randomized, as illustrated in figure 3b.

It is noted that in figure 3b, the filling level FLV exceeds the carrier material zone CMZ. This illustrates that the carrier material zone CMZ is defined by the carrier material CM when the packed bed reactor PBR is empty, i.e. not filled with aqueous solution AS.

Finally, the aqueous solution AS is removed from the packed bed reactor PBR by establishing a flow though the aqueous solution outlet ASO. The emptied packed bed reactor PBR is illustrated in figure 3c. In some embodiments, an amount of aqueous solution may be left in the lower part of the packed bed reactor PBR, below the carrier material zone.

As illustrated in figure 3c, the configuration of the carrier material CM is different from compared to before the filling and removing steps, as illustrated by the different orientation of the carrier material CM in figures 3a and 3c.

It is noted that conversion of carbon dioxide from the input gas to methane may be carried out before and/or after the filling and removing sequence illustrated in figures 3a-3c.

Now referring to figure 4, an embodiment of the invention is illustrated, which is combinable with the embodiment of figure 1.

As illustrated in figure 4, filling level FLV exceeds the carrier material zone CMZ. This illustrates that in some embodiments, the packed bed reactor PBR may be nearly or completely filled with the aqueous solution AS. As illustrated in figure 4, a remaining volume RV is defined by the part of the packed bed reactor PBR not filled with aqueous solution AS. For example, with a filling level of 95% by volume of the packed bed reactor PBR, the remaining volume RV would be 5% by volume. In some embodiments, the remaining volume RV may be very small or even eliminated by completely filling the packed bed reactor PBR.

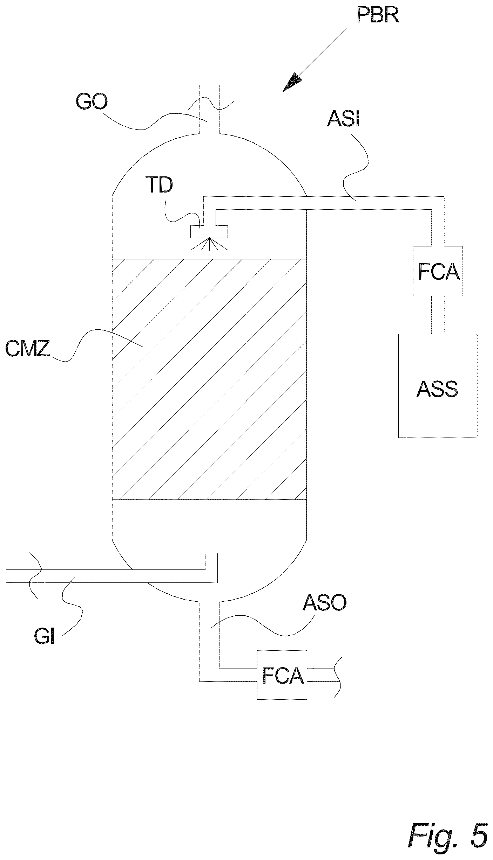

Now referring to figure 5, a system for producing methane according to an embodiment of the invention is illustrated. The system is combinable with the embodiment of figure 1.

The system comprises a packed bed reactor PBR comprising a carrier material for microorganisms, where the carrier material defines a carrier material zone CMZ, as e.g. illustrated in figure 3a. The system further comprises an aqueous solution source ASS connected to the packed bed reactor, a flow controlling arrangement FCA, a hydrogen gas inlet GI connected to the packed bed reactor, and an input gas inlet GI connected to the packed bed reactor.

It is noted that the flow controlling arrangement FCA may comprise one or more valves and/or a pump arrangement. It is also noted that the input gas inlet and the hydrogen gas inlet are illustrated in figure 5 as a common gas inlet GI for both the hydrogen gas and the input gas, but these may in other embodiments be separate inlets for the hydrogen gas in the input gas. It is also noted that the input gas comprises carbon dioxide.

The system further comprises a gas outlet GO for removing produced methane from the packed bed reactor, and also any unconverted input gas.

In figure 5, the flow controlling arrangement FCA is configured to establish and control a flow of aqueous solution from said aqueous solution source ASS into said packed bed reactor, through an aqueous solution inlet ASI. Similarly, it is configured to establish and control a flow of aqueous solution out from said packed bed reactor through an aqueous solution outlet ASO. Thereby, the flow controlling arrangement FCA can control filling and removing aqueous solution into the packed bed reactor PBR, e.g. as illustrated in figures 2a-2b as well as 3a-3c and 4. In figure 5, the flow controlling arrangement FCA comprises two separate units, however, in other embodiments, the flow controlling arrangement FCA may be provided as a single unit.

In figure 5, the flow aqueous solution inlet ASI comprises a trickling dispenser TD for continuously or periodically ejecting aqueous solution over the carrier material in the carrier material zone CMZ.

In some embodiments the trickling dispenser TD may be used for filling the packed bed reactor PBR up to the desired filling level. In such embodiment, the same aqueous solution may be used for the filling step and in the trickling, i.e. for continuously or periodically ejecting aqueous solution over the carrier material.

In some embodiments, the system may comprise a separate aqueous solution inlet for feeding aqueous solution in the filling step, e.g. as illustrated figures 3a-3c, in addition to the trickling dispensers. Such separate aqueous solution inlet may be positioned in the bottom of the reactor. In such embodiments, the same aqueous solution or different aqueous solutions may be used for filling and trickling.

### REFERENCE

### List of reference signs:

- AS: Aqueous solution
- ASI: Aqueous solution inlet
- ASO: Aqueous solution outlet
- ASS: Aqueous solution source
- CMZ: Carrier material zone
- FLV: Filling level
- FCA: Flow controlling arrangement
- GFR: Gas fermentation reactor
- GI: Gas inlet
- GO: Gas outlet
- HG: Hydrogen gas
- IG: Input gas
- MET: Methane
- PBR: Packed bed reactor
- RV: Remaining volume
- TD: Trickling dispenser

## Claims

1. A method for producing methane (MET) comprising the steps of
providing an aqueous solution (AS),
providing a packed bed reactor (PBR) comprising a non-polymeric carrier material for microorganisms, the carrier material defining a carrier material zone (CMZ),
providing a hydrogen gas (HG) and an input gas (IG) comprising carbon dioxide to the packed bed reactor (PBR) to convert at least part of the carbon dioxide to methane (MET), and
filling at least 10% by volume of said carrier material zone (CMZ) with said aqueous solution (AS) followed by removing at least part of said aqueous solution (AS) from the carrier material zone (CMZ).

2. The method according to claim 1, wherein the step of filling and removing is before the step of converting carbon dioxide to methane (MET).

3. The method according to claim 1 or 2, wherein the step of filling and removing is after the step of converting carbon dioxide to methane (MET).

4. The method according to any of claims 1-3, wherein said aqueous solution (AS) is selected from the group consisting of wastewater, digestate, agricultural residues such as manure components, synthetic mediums, aqueous fertilizer, water such as microbially produced metabolic water, and any combination thereof.

5. The method according to any of claims 1-4, wherein said packed bed reactor (PBR) comprises trickling.

6. The method according to any of claims 1-5, wherein said aqueous solution (AS) comprises microorganisms, such as microorganisms for biogas and/or biomethane production.

7. The method according to any of claims 1-6, wherein the carrier material is an inorganic material.

8. The method according to any of claims 1-7, wherein the carrier material is floatable.

9. The method according to any of claims 1-8, wherein the input gas (IG) comprises raw biogas.

10. The method according to any of claims 1-9, wherein said flow of the aqueous solution (AS) into the packed bed reactor (PBR) is configured to fill at least 90% by volume of said packed bed reactor (PBR) with said aqueous solution (AS), such as at least 95% by volume of said packed bed reactor (PBR), such as at least 98% by volume of said packed bed reactor (PBR), such as 100% by volume of said packed bed reactor (PBR).

11. The method according to any of claims 1-10, wherein filling and removing step comprises removing at least 5% of said aqueous solution (AS) from the carrier material zone (CMZ), such as at least 15% from said carrier material zone (CMZ), such as at least 30% from said carrier material zone (CMZ), such as at least 50% from said carrier material zone (CMZ), such as at least 75% from said carrier material zone (CMZ), such as 100% from said carrier material zone (CMZ).

12. The method according to any of claims 1-11, wherein the step of filling and removing reduces the content of impurities in the packed bed reactor (PBR).

13. The method according to any of claims 1-12, wherein said carrier material is porous.

14. The method according to any of claims 1-13, wherein the packed bed reactor has an interior atmosphere comprising oxygen before or after the step of filling and removing.

15. A system for producing methane (MET), the system comprising
- a packed bed reactor (PBR) comprising a non-polymeric carrier material for microorganisms, the carrier material defining a carrier material zone (CMZ),
- an aqueous solution source (ASS) connected to the packed bed reactor (PBR),
- a flow controlling arrangement (FCA) such as a pump arrangement,
- a hydrogen gas inlet (GI) connected to the packed bed reactor (PBR), and
- an input gas inlet (GI) connected to the packed bed reactor (PBR), the input gas (IG) comprising carbon dioxide,
wherein the flow controlling arrangement (FCA) is configured to establish a flow of aqueous solution (AS) from said aqueous solution source (ASS) into said packed bed reactor (PBR) to fill at least 10% by volume of the carrier material zone (CMZ), and
to establish a flow of aqueous solution (AS) from said packed bed reactor (PBR) to remove at least part of said aqueous solution (AS) from the carrier material zone (CMZ), and
wherein the packed bed reactor (PBR) is configured to convert at least part of the carbon dioxide to methane (MET).
